# EUROPÄISCHE PATENTSCHRIFT

(11) **EP 3 139 870 B1**
(45) Veröffentlichungstag und Bekanntmachung des Hinweises auf die Patenterteilung: **18.12.2019**
(21) Anmeldenummer: 15723431.1
(22) Anmeldetag: 05.05.2015
(51) Int. Cl.: A61F 2/50, A61F 2/66, A43B 7/14, B29C 65/54, B29C 65/78, B29L 31/50

(54) **VERFAHREN ZUM VERBINDEN ZUMINDEST ZWEIER STRUKTURBAUTEILE EINER ORTHOPÄDIETECHNISCHEN KOMPONENTE UND ORTHOPÄDIETECHNISCHE KOMPONENTE MIT ZUMINDEST ZWEI STRUKTURBAUTEILEN**
METHOD FOR CONNECTING AT LEAST TWO STRUCTURAL PARTS OF AN ORTHOPEDIC COMPONENT AND ORTHOPEDIC COMPONENT HAVING AT LEAST TWO STRUCTURAL PARTS
PROCÉDÉ DE LIAISON D'AU MOINS DEUX ÉLÉMENTS STRUCTURAUX D'UN COMPOSANT ORTHOPÉDIQUE ET COMPOSANT ORTHOPÉDIQUE COMPRENANT AU MOINS DEUX ÉLÉMENTS STRUCTURAUX

(30) Priorität: 07.05.2014 DE 102014006570
(43) Veröffentlichungstag der Anmeldung: 15.03.2017
(73) Patentinhaber: Ottobock SE & Co. KGaA, 37115 Duderstadt (DE)
(72) Erfinder: MÖNICKE, Carsten, 37115 Duderstadt (DE); RANE, Darshan, Milcreek, UT 84106 (US); STEPHENSON, Tony, North Salt Lake, UT 84054 (US)
(74) Vertreter: Gramm, Lins & Partner Patent- und Rechtsanwälte PartGmbB
(86) Internationale Anmeldenummer: PCT/EP2015/000917
(87) Internationale Veröffentlichungsnummer: WO 2015/169438

(56) Entgegenhaltungen:
- EP-A1- 0 043 874
- US-A- 3 510 968
- US-A- 3 812 604

## Beschreibung

Die Erfindung betrifft ein Verfahren zum Verbinden zumindest zweier Strukturbauteile einer orthopädietechnischen Komponente, bei dem die Strukturbauteile in einer Ausrichteeinrichtung unter Ausbildung eines Zwischenraums zwischen den Strukturbauteilen zueinander orientiert gehalten werden. Die Erfindung betrifft ebenfalls eine orthopädietechnische Komponente mit zumindest zwei Strukturbauteilen, die zueinander beabstandet miteinander verklebt sind, wobei die Strukturbauteile in einer Ausrichteeinrichtung zueinander orientiert gehalten sind, und die Ausrichteeinrichtung Abstandhalter aufweist, die Strukturbauteile voneinander beabstanden.

Ein derartiges Verfahren ist aus der US 3812604 und der EP 0043874 A1 bekannt.

Strukturbauteile, insbesondere solche aus vorgefertigten Halbzeugen auf Basis faserverstärkter Kunststoffe, können auf verschiedene Arten und Weisen miteinander verbunden werden. Neben einem Verschrauben, bei dem Durchgangslöcher durch beide Strukturbauteile hindurch eingebracht oder eingearbeitet werden, durch die Schraubbolzen gesteckt und miteinander verbunden werden, besteht die Möglichkeit, dass die beiden Strukturbauteile miteinander verklebt werden. Dazu werden die Bauteile zueinander ausgerichtet, in der ausgerichteten Form zueinander gehalten und ein Zwischenraum, der zwischen den Strukturbauteilen ausgebildet ist, wird mit einem Klebstoff befüllt. Die Zuordnung der beiden Bauteile wird solange aufrechterhalten, wie es dauert, bis der Klebstoff ausreichend ausgehärtet ist. Anschließend werden die gegebenenfalls vorgesehenen Halteeinrichtungen entfernt und der regelmäßig über die Klebestelle hinaus ausgetretene Klebstoff wird im Rahmen einer Nacharbeit entfernt. Diese Nacharbeit ist mühevoll und kann nur in zeitaufwändiger Handarbeit ausgeführt werden. Aufgabe der vorliegenden Erfindung ist es, ein Verfahren zum Verbinden zumindest zweier Strukturbauteile einer orthopädietechnischen Komponente sowie eine orthopädietechnische Komponente als solche bereitzustellen, die kostengünstiger auszuführen sind, eine präzise Ausrichtung der Strukturbauteile zueinander gewährleisten und den Aufwand an Nacharbeit verringern.

Erfindungsgemäß wird diese Aufgabe durch ein Verfahren mit den Merkmalen des Hauptanspruches und eine orthopädietechnische Komponente mit den Merkmalen des nebengeordneten Anspruchs 7 gelöst. Vorteilhafte Ausgestaltungen und Weiterbildungen der Erfindung sind in den Unteransprüchen, in der Beschreibung sowie den Figuren offenbart.

Das Verfahren zum Verbinden zumindest zweier Strukturbauteile einer orthopädietechnischen Komponente, bei dem die Strukturbauteile in einer Ausrichteeinrichtung unter Ausbildung eines Zwischenraumes zwischen den Strukturbauteilen zueinander orientiert gehalten werden, sieht vor, dass die Ausrichteeinrichtung und die Strukturbauteile gemeinsam einen Hohlraum ausbilden, der mit zumindest einem Zuführanschluss in strömungstechnischer Verbindung steht und über den ein Klebstoff zum Verkleben der Strukturbauteile in den Hohlraum eingeführt wird, wobei die Ausrichteeinrichtung nach dem Verkleben nicht von den Strukturbauteilen getrennt wird.

Durch die Ausrichteeinrichtung, die als eine sogenannte Formenhülle ausgebildet ist und an dem Strukturbauteil verbleibt, ist es möglich, neben einer Ausrichtung der Strukturbauteile zueinander auch einen Hohlraum zu bilden, der im Wesentlichen abgeschlossen ist und in der Regel nur einen Zuführanschluss aufweist, beispielsweise eine Bohrung in einer Seitenwand, durch die der Klebstoff in den Hohlraum eingeführt wird. Der Hohlraum wird zumindest teilweise durch die Strukturbauteile begrenzt, so dass der eingeführte Klebstoff in Kontakt mit den Strukturbauteilen tritt und diese miteinander verkleben kann. Der Hohlraum weist bevorzugt noch einen Auslasskanal auf, so dass beim Einführen des Klebstoffes in den Hohlraum die Luft verdrängt werden kann, so dass der Hohlraum vollständig ausgefüllt werden kann. Sobald Klebstoff aus dem Auslasskanal austritt, ist sichergestellt, dass der Hohlraum vollständig mit Klebstoff befüllt ist, die Befüllung mit Klebstoff wird beendet, der Zuführanschluss und Auslasskanal verschlossen und abgewartet, bis der Klebstoff ausgehärtet ist. Auf diese Weise wird einerseits die Zuordnung der Strukturbauteile zueinander reproduzierbar und präzise zueinander hergestellt und andererseits verhindert, dass Klebstoff außerhalb des Hohlraumes in Kontakt mit den Strukturbauteilen tritt, wodurch Nacharbeit vermieden wird. Der Klebstoff verfügt vorzugsweise im ausgehärteten Zustand über elastische Eigenschaften, um beispielsweise bei einer Fußprothese bei dem Abrollvorgang nicht zu reißen oder zu brechen. Vorzugsweise werden als Klebstoffe PU, TPU und/oder TPE eingesetzt. Für starre Verbindungen in Bereichen, die einer geringeren oder unkritischen dynamischen Belastung ausgesetzt sind, können auch Harze eingesetzt werden, was insbesondere bei faserverstärkten vorteilhaft ist, da die gleichen Harze verwendet werden können, die zur Bildung der Matrix der Fasern eingesetzt werden, wodurch sich eine gute Verbindung der Strukturbauteile mit dem Klebstoff ergibt.

Die Strukturbauteile werden solange zueinander orientiert gehalten, bis der Klebstoff ausgehärtet ist. Dies kann beispielsweise dadurch geschehen, dass in der Ausrichteeinrichtung Aufnahmen, Schlitze und Führungen angeordnet oder ausgebildet sind, so dass die Ausrichteeinrichtung selbsttätig an den Strukturbauteilen festgelegt wird. Alternativ besteht die Möglichkeit, dass über eine Klemmeinrichtung oder eine sonstige Fixiereinrichtung die Strukturbauteile an der Ausrichteeinrichtung fixiert werden, beispielsweise durch eine Klemme, so dass auch bei Zufuhr des Klebstoffes unter Druck die Orientierung der Strukturbauteile zueinander nicht aufgehoben wird. In einer besonderen Ausgestaltung kann eine zusätzliche Ausrichtung der Strukturbauteile in einer Ebene auch über eine Vorrichtung oder eine Zusatzeinrichtung erfolgen. Während die Ausrichteeinrichtung die Position der Strukturbauteile in einer Ebene und beispielsweise an einem Ende der orthopädietechnischen Komponente sicherstellt, kann die Zusatzeinrichtung oder Vorrichtung die Ausrichtung in einer anderen Ebene oder Orientierung sicherstellen, so dass die Ausrichteeinrichtung nicht ausschließlich die Positionierung der Strukturbauteile zueinander bewirkt. Die Zusatzeinrichtung stellt die Ausrichtung in einer anderen Ebene oder Orientierung sicher, beispielsweise kann auf einen Rand verzichtet werden und die Orientierung und Positionierung der freien Enden der Strukturbauteile erfolgt über die Zusatzeinrichtung, beispielsweise die Presse oder eine Klemmeinrichtung.

Vorteilhafterweise wird zumindest ein Strukturbauteil in dem Klebstoff eingebettet, wobei keine vollständige Einbettung in dem Klebstoff erfolgen muss, vielmehr reicht es aus, wenn zumindest ein Strukturbauteil an mehr als einer Oberfläche, beispielsweise an einer Oberseite und einer Unterseite oder an mehreren Seitenflächen in Kontakt mit dem Klebstoff tritt, um eine mehrseitige Verklebung des Strukturbauteils zu bewirken. Grundsätzlich ist ausreichend, wenn nur eine Oberfläche des jeweiligen Strukturbauteils, beispielsweise eine Oberseite, Seitenfläche oder Unterseite eines blattfederartigen oder stegartigen Bauteils mit dem Klebstoff in Verbindung tritt, um eine Verklebung mit dem anderen Strukturbauteil und der Ausrichteeinrichtung zu bewirken.

Eine Weiterbildung der Erfindung sieht vor, dass während des Verklebens zumindest ein Strukturbauteil gegen die Ausrichteeinrichtung gepresst wird, wodurch es nicht notwendig ist, die Ausrichteeinrichtung als ein selbstständig abdichtendes, die Strukturbauteile zueinander zuordnendes Formteil auszubilden. So ist es ausreichend, dass über Distanzelemente und einen umlaufenden Rand ein Bereich oberhalb eines ersten Strukturbauteils umgeben wird, der auf der Oberfläche des ersten Strukturbauteils aufliegt und nach oben offen ist. Diese nach oben offene Aufnahme, die beispielsweise Führungsränder für das zweite Strukturbauteil aufweisen kann, wird durch das zweite Strukturbauteil verschlossen, indem das zweite Strukturbauteil auf die Ausrichteeinrichtung aufgelegt und in Richtung auf das erste Strukturbauteil mit einer Kraft beaufschlagt wird.

Der Hohlraum wird durch das Halten der Strukturbauteile abgedichtet, selbstverständlich unter Aufnahme des Zuführanschlusses und des Auslasskanals, so dass ein im Wesentlichen abgeschlossener Hohlraum entsteht, aus dem der zugeführte Klebstoff nur durch den Auslasskanal entweichen kann. Durch die nahezu vollständige Abdichtung des Hohlraumes oder der Hohlräume ist es möglich, Nacharbeiten zu vermeiden oder auf ein Minimum zu reduzieren.

Der Klebstoff kann über Zuführeinrichtungen, beispielsweise Zuführschläuche, in den Hohlraum eingeführt werden, wobei die Zuführeinrichtungen und gegebenenfalls auch Auslasseinrichtungen wie Auslassschläuche, bis zum Aushärten an der Ausrichteeinrichtung verbleiben und nach dem Aushärten entfernt werden. Die Zuführeinrichtungen und gegebenenfalls Auslasseinrichtungen wie Schläuche, Röhren und dergleichen, können als separate Komponenten ausgebildet sein, die in die jeweiligen Kanäle eingesteckt werden und die verhindern, dass während des Aushärtens Klebstoff in direkten Kontakt mit der Oberfläche sowohl der Strukturbauteile als auch der Ausrichteeinrichtung gelangt. Durch Herausziehen der Zuführeinrichtungen und der Auslasseinrichtungen ist es möglich, eine saubere Verbindung ohne Nacharbeiten und ohne die Gefahr von Klebstoffresten auf den jeweiligen Komponenten herzustellen.

Die orthopädietechnische Komponente mit zumindest zwei Strukturbauteilen, die zueinander beabstandet miteinander verklebt sind, sieht vor, dass die Strukturbauteile in einer Ausrichteeinrichtung zueinander orientiert gehalten sind und die Ausrichteeinrichtung zumindest einen Abstandshalter aufweist, der die Strukturelemente voneinander beabstandet. Durch den Abstandshalter oder die Abstandshalter ist es möglich, einen Hohlraum zwischen den Strukturbauteilen auszubilden, der durch den Klebstoff gefüllt, vorteilhafterweise vollständig ausgefüllt wird, so dass eine möglichst großflächige Verklebung der Strukturbauteile zueinander gewährleistet ist.

Die Ausrichteeinrichtung wird mit den Strukturbauteilen verklebt und verbleibt nach dem Verkleben an den Strukturbauteilen, so dass die Ausrichteeinrichtung Teil der orthopädietechnischen Komponente wird. Die Ausrichteeinrichtung dient somit einerseits als Form, andererseits als funktionale Komponente, z.B. zum Schutz, so dass die Ausrichteeinrichtung als Formhülle bezeichnet werden kann, die einerseits eine verlorene Form darstellt und andererseits eine zusätzliche Funktion ausübt, nämlich den Schutz der Strukturbauteile durch eine entsprechende Materialwahl und/oder als Abrollkontur durch seine unterseitige Formgebung. Die Ausrichteeinrichtung ist vorteilhafterweise aus einem flexiblen, elastischen Kunststoff hergestellt, der die Strukturelemente zumindest teilweise umgibt, so dass durch die Ausrichteeinrichtung auch funktionelle Anforderungen erfüllt werden, die durch die Strukturbauteile als solche, beispielsweise aufgrund der Materialbeschaffenheit, nicht erfüllt werden können. Beispielsweise ist es möglich, dass die Ausrichteeinrichtung eine Polsterfunktion und eine Profilierfunktion aufweist, so dass zusätzlich eine Formgebung der Strukturbauteile durch die Ausrichteeinrichtung wahrgenommen werden kann.

Die Ausrichteeinrichtung weist zumindest eine Einführöffnung für zumindest ein Strukturelement auf, wobei die Form der Einführöffnung der Kontur des Strukturbauteils im Wesentlichen entspricht. Die Einführöffnung ermöglicht die Zuordnung des Strukturbauteils zu der Ausrichteeinrichtung, wenn die Ausrichteeinrichtung auf das Strukturelement aufgesteckt bzw. das Strukturelement in die Ausrichteeinrichtung eingeführt wird. Innerhalb der Ausrichteeinrichtung sind Führungselemente oder -einrichtungen vorgesehen, beispielsweise Schlitze, Nuten oder Vorsprünge, so dass die Ausrichteeinrichtung definiert an dem Strukturbauteil festgelegt ist. Die Einführöffnung selbst liegt möglichst eng an der Kontur des Strukturbauteils an, um beim Befüllen des Hohlraums mit Klebstoff ein ungewolltes Austreten von Klebstoff zu vermeiden.

Vorteilhafterweise sind die Strukturbauteile als Blattfedern ausgebildet, die parallel zueinander geschaltet sind und in dem Bereich des Hohlraumes in Kontakt mit dem Klebstoff treten, so dass dort eine stabile und großflächige Verklebung der Blattfedern miteinander vorliegt.

Zumindest ein Strukturbauteil ist vorzugsweise mehrseitig von dem Klebstoff umgeben, um eine möglichst definierte und stabile Klebeverbindung zwischen den Strukturbauteilen und der Ausrichteeinrichtung zu gewährleisten.

In der Ausrichteeinrichtung ist vorteilhafterweise zumindest ein Verbindungskanal ausgebildet, der zwei voneinander durch ein Strukturbauteil getrennte Hohlräume miteinander verbindet. So kann es möglich sein, dass an der Unterseite des Strukturbauteils ein Hohlraum ausgebildet ist, der zwischen dem Strukturbauteil und der Ausrichteeinrichtung gebildet wird. Durch diesen unteren Hohlraum kann beispielsweise der Klebstoff eingeleitet werden, so dass es notwendig ist, von dem unterseitigen Hohlraum der Klebstoff in den Hohlraum zu leiten, der zwischen den Strukturbauteilen gebildet wird. Von dort kann wieder ein Verbindungskanal zu entweder dem Auslasskanal, der an der vorteilhafterweise geodätisch oberen oder höchsten Stelle angeordnet ist, führen, um die Luft vollständig aus den Hohlräumen herauszudrücken, wenn der Klebstoff eingeführt wird. Ebenso ist es möglich, dass mehr als zwei Strukturbauteile zueinander orientiert verklebt werden sollen, so dass jeweils zwischen zwei Strukturbauteilen ein Hohlraum vorhanden ist, bei drei Strukturbauteilen somit zumindest zwei Hohlräume, die miteinander verbunden werden müssen, um zu vermeiden, dass mehrere Zuführanschlüsse zum Befüllen des Hohlraumes oder der Hohlräume vorgesehen werden müssen. Grundsätzlich besteht auch die Möglichkeit, bei mehreren voneinander getrennten Hohlräumen jeden oder mehrere mit einem Zuführanschluss und einem entsprechenden Auslasskanal zu versehen.

Die orthopädietechnische Komponente kann als Prothesenfuß oder Orthesenkomponente ausgebildet sein.

Die Ausrichteeinrichtung kann an zumindest einem Strukturbauteil an zumindest drei Seiten anliegen oder es umgeben. Bei einer flächigen Ausgestaltung des Strukturbauteils und einem Auflegen und Einlegen in einen Rahmen ist in der Regel der Rahmen an der Vorderseite und den beiden seitlichen Kanten des Strukturbauteils angeordnet. Wird ein Strukturbauteil in einen Schlitz eingefügt, liegt die Ausrichteeinrichtung zumindest teilweise an der Unterseite, der Oberseite, der Vorderseite und den beiden Seitenflächen an und ragt aus der Einführöffnung heraus.

Das Strukturbauteil ist bevorzugt als faserverstärktes Kunststoffbauteil ausgebildet, grundsätzlich ist es möglich, auch andere Strukturbauteile miteinander zu verkleben und über eine solche Klebeverbindung zusammen mit der Ausrichteeinrichtung aneinander zu fixieren. Ebenso ist es möglich, dass Strukturbauteile unterschiedlicher Materialien, beispielsweise Metalle und Faserverbundwerkstoffe miteinander über eine solche Einrichtung verklebt werden, da kein unmittelbarer Kontakt zwischen den Strukturbauteilen notwendig ist und die beabstandete Zuordnung der Strukturbauteile unter Zwischenschaltung des Klebstoffes eine beabstandete Befestigung der Strukturbauteile aneinander gewährleistet. Über die Ausrichteeinrichtung wird zudem eine Schutzummantelung, zumindest eine teilweise Schutzummantelung bereitgestellt.

Nachfolgend werden Ausführungsbeispiele der Erfindung anhand der beigefügten Figuren näher erläutert. Es zeigen:
- Figur 1: eine Seitenansicht einer orthopädietechnischen Komponente während der Fertigung;
- Figur 2: eine perspektivische Ansicht mit Zuführ- und Auslasseinrichtungen;
- Figur 3: eine Schnittansicht eines Teils einer orthopädietechnischen Komponente;
- Figur 4: eine perspektivische Ansicht einer Ausrichteeinrichtung;
- Figur 5: eine andere Ansicht der Ausrichteeinrichtung aus Figur 4;
- Figur 6: eine Teildarstellung einer orthopädietechnischen Komponente von schräg hinten;
- Figur 7: eine Variante der Figur 6;
- Figur 8: eine perspektivische Teildarstellung einer zweiten Ausführungsform;
- Figur 9: eine andere Ansicht der Ausführungsform aus Figur 8;
- Figur 10: eine Gesamtansicht einer Ausrichteeinrichtung der zweiten Ausführungsform;
- Figur 11: eine Schnittdarstellung der Figur 10;
- Figur 12: eine Schnittdarstellung der Figur 9;
- Figur 13: eine andere Ansicht der Figur 12 sowie
- Fig. 14: eine Variante der Ausrichteeinrichtung ohne Boden.

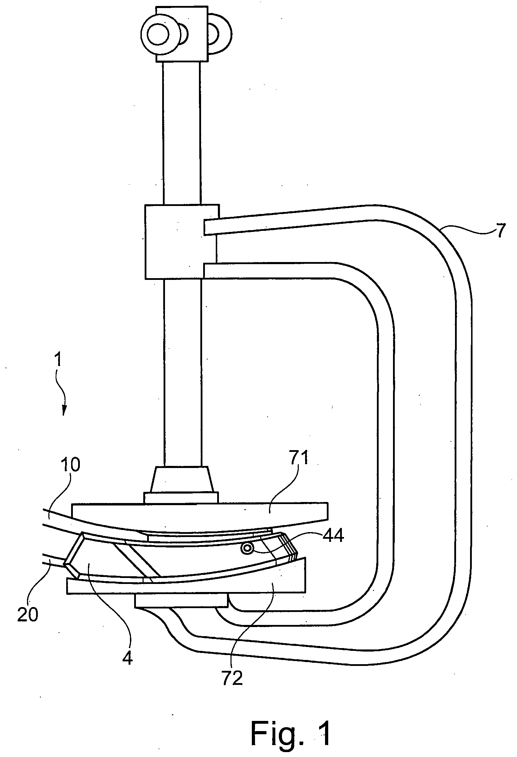

Figur 1 zeigt in einer Seitenansicht eine schematische Darstellung eines vorderen Teils einer orthopädietechnischen Komponente 1, die in Gestalt eines Prothesenfußes ausgebildet ist. Der Prothesenfuß weist zwei Strukturbauteile 10, 20 auf, die als Blattfedern aus einem faserverstärkten Kunststoff hergestellt sind. Es ist der Vorderfußbereich der orthopädietechnischen Komponente 1 dargestellt, das erste Strukturbauteil 10 ist eine Vorderfußfeder, das zweite Strukturbauteil 20 ist eine Basisfeder. Die Vorderfußfeder 10 erstreckt sich schräg nach oben zu einem oberen Anschlusspunkt, an dem Befestigungseinrichtungen oder Anschlussmittel zur Befestigung an einem Unterschenkelrohr oder einem Unterschenkelschaft befestigbar sind. Die Basisfeder 20 führt bis in den Fersenbereich, wobei sich eine Fersenfeder von der Basisfeder 20 zur Vorfußfeder 10 und/oder den oberen Anschlussmitteln erstrecken kann.

Die Strukturbauteile 10, 20 sind einer Ausrichteeinrichtung 4 zugeordnet, die als Kunststoffspritzgussteil ausgebildet ist. Die Ausrichteeinrichtung 4 kann aus einem Polyurethan, einem technischen Polyethylen, einem technischen Polyurethan, Gummi oder einem anderen Kunststoff, vorzugsweise Elastomer, bestehen. Die Ausrichteeinrichtung 4 weist einen Einführschlitz für das zweite Strukturbauteil 20 sowie einen Aufnahmebereich an der Oberseite für das erste Strukturbauteil 10 auf, auf den das erste Strukturbauteil 10 aufgelegt werden kann. Der Auflagebereich ist von Wänden umrahmt, so dass das erste Strukturbauteil 10 in einer definierten Position zu der Ausrichteeinrichtung 4 aufgelegt werden kann, wenn sich die Kontur des Strukturbauteils 10 an die Wände um den Auflagebereich herum anlegt.

Das zweite Strukturbauteil 20 wird in einen nicht dargestellten Schlitz innerhalb der Ausrichteeinrichtung eingeführt, so dass die Unterseite des zweiten Strukturbauteils 20 oder der Blattfeder von einer geschlossenen Oberfläche der Unterseite der Ausrichteeinrichtung 4 abgedeckt ist. Zwischen den beiden Strukturbauteilen 10, 20 ist ein Abstandshalter ausgebildet, der die beiden Strukturbauteile 10, 20 beabstandet zueinander festhält. Durch das Einführen des zweiten Strukturbauteils 20 in die Ausrichteeinrichtung 4 ist auch dieses Strukturbauteil 20 definiert zugeordnet, beispielsweise indem das zweite Strukturbauteil 20 in einem Schlitz oder in einer Nut innerhalb der Aufnahmeeinrichtung 4 geführt ist. Dadurch bilden die beiden Strukturbauteile 10, 20 und die Aufnahmeeinrichtung 4 einen Hohlraum aus, der im Wesentlichen geschlossen ist. In einer Seitenwand der Ausrichteeinrichtung 4 ist ein Zuführanschluss 44 vorgesehen, der in strömungstechnischer Verbindung mit dem nicht dargestellten Hohlraum steht und durch den Klebstoff in den Hohlraum eingeführt bzw. hineingepumpt werden kann. Auf der dem Zuführanschluss 44 abgewandten Seite ist ein Auslasskanal vorgesehen, der ebenfalls in strömungstechnischer Verbindung mit dem Hohlraum steht, so dass die innerhalb des Hohlraumes befindliche Luft austreten kann und der Hohlraum vollständig mit Klebstoff befüllt werden kann.

Die Strukturbauteile 10, 20 und die Ausrichteeinrichtung 4 sind in einer Presse 7, die als übliche Schraubzwinge ausgebildet sein kann, gehalten. An der Presse 7 sind zwei Pressenschuhe 71, 72 angeordnet, die eine Kontur aufweisen, die der jeweilig zugeordneten Kontur der orthopädietechnischen Komponente 1 entspricht. Im dargestellten Ausführungsbeispiel ist der obere Pressenschuh 71 mit einer konvexen Wölbung und der untere Pressenschuh 72 mit einer konkaven Wölbung ausgestattet, so dass einerseits die Unterseite der Aufnahmeeinrichtung 4 und andererseits die Oberseite des ersten Strukturbauteils 10 vollflächig an der Oberfläche des jeweiligen Pressenschuhs 71, 72 anliegen können. Wird die Presse 7 geschlossen und Druck auf die Pressenschuhe 71, 72 ausgeübt, wird das erste Strukturbauteil 10 auf die Oberfläche der Auflagefläche an der Ausrichteeinrichtung 4 gepresst, so dass der zwischen den Strukturbauteilen 10, 20 oben und unten und an den Seitenflächen durch die Ausrichteeinrichtung 4 gebildete Hohlraum abgeschlossen ist und eine Zufuhr von Klebstoff nur durch den Zuführanschluss 44 und ein Austreten von Luft und gegebenenfalls überschüssigen Klebstoff durch den Auslasskanal erfolgen kann.

Nach dem Einführen des Klebstoffes wird der Pressdruck aufrechterhalten, bis der Klebstoff ausgehärtet ist, so dass eine dauerhafte Verbindung zwischen dem ersten Strukturbauteil 10, dem zweiten Strukturbauteil 20 und der Ausrichteeinrichtung 4 erzielt wird. Nach Aushärtung des Klebstoffes verbleibt die Ausrichteeinrichtung 4 an der orthopädietechnischen Komponente 1 und dient einerseits als Schutz für die Strukturbauteile 10, 20 und andererseits als funktionale Komponente der orthopädietechnischen Komponente, beispielsweise als Formgebung für den Prothesenfuß, als Polster, als Sohlenstruktur oder bei anderen Ausgestaltungsformen als Aufnahmeeinrichtung oder Schutzeinrichtung für weitere Komponenten.

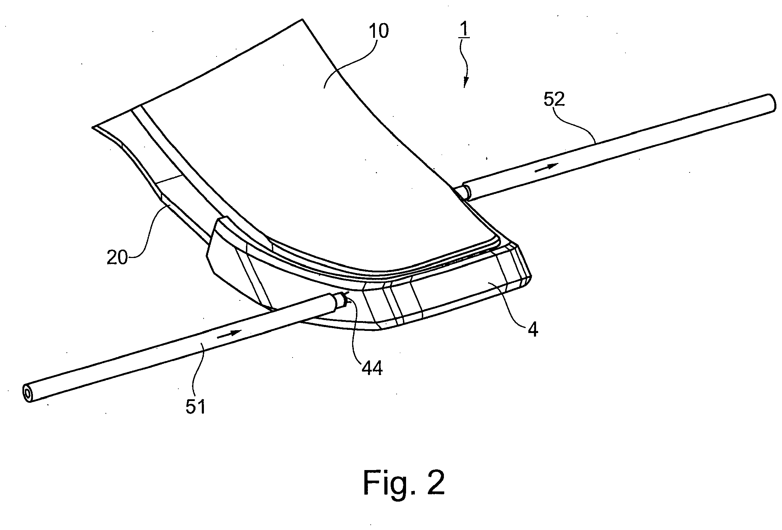

Figur 2 zeigt in einer perspektivischen Schrägdraufsicht die Fertigung der orthopädietechnischen Komponente 1, zumindest das Verbinden der Strukturbauteile 10, 20 mit der Ausrichteeinrichtung 4. An der Ausrichteeinrichtung 4 ist an dem Zuführanschluss 44 eine Zuführeinrichtung 51 angebracht, die im dargestellten Ausführungsbeispiel als Schlauch oder Rohr ausgebildet ist, und durch die Klebstoff, wie durch den Pfeil angedeutet, in den nicht dargestellten Hohlraum eingeleitet wird. Der Hohlraum ist an der Oberseite und an der Unterseite durch die Strukturbauteile 10, 20, an der Vorderseite und an den Seitenkanten durch die Seitenwände der Ausrichteeinrichtung 4 und an der Rückseite zwischen den Strukturbauteilen 10, 20 durch einen Abstandshalter, der dichtend sowohl an der Unterseite des ersten Strukturbauteils 10 als auch an der Oberseite des zweiten Strukturbauteils 20 anliegt, gebildet und abgeschlossen. In der Figur 2 ist die Presse 7 nicht dargestellt, während der Zufuhr des Klebstoffes wird jedoch die Zuordnung der jeweiligen Komponenten 4, 10, 20 durch die Presse 7 oder eine andere, geeignete Fixiereinrichtung aufrechterhalten.

Klebstoff wird durch die Zuführeinrichtung 51 und den Zuführanschluss 44 in den Hohlraum eingeführt, die in dem Hohlraum befindliche Luft wird durch den Klebstoff verdrängt und durch eine Auslasseinrichtung 52 abtransportiert. Die Auslasseinrichtung 52 ist an einem nicht dargestellten Auslasskanal, der strömungstechnisch in Verbindung mit dem Hohlraum innerhalb der Aufnahmeeinrichtung 4 steht, verbunden, so dass Luft und gegebenenfalls überschüssiger Klebstoff aus dem Auslasskanal durch die Auslassöffnung 51 austreten kann, wie durch den Pfeil angedeutet. Sowohl der Zuführanschluss 44 als auch der Auslasskanal sind bevorzugt in einem Abstandshalter angeordnet, der sicherstellt, dass die Strukturbauteile 10, 20 zueinander beabstandet gehalten werden. Dadurch wird sichergestellt, dass durch die Anordnung der Strukturbauteile 10, 20 an oder in der Ausrichteeinrichtung 4 der Zuführanschluss 44 und der Auslasskanal versperrt werden.

Die nicht dargestellte Presse 7 hält die Zuordnung der Komponenten 4, 10, 20 zueinander, solange der Klebstoff aushärtet. Nach dem Aushärten werden die Zuführeinrichtung 51 und die Auslasseinrichtung 52 von der Ausrichteeinrichtung 4 getrennt, beispielsweise abgeknickt, so dass ein nahezu glatter Abschluss der Ausrichteeinrichtung 4 im Bereich des Zuführanschlusses 44 und des Auslasskanals erreicht werden kann. Dies kann beispielsweise durch eine Sollbruchstelle an der Zuführeinrichtung 51 und/oder der Auslasseinrichtung 52 im Bereich des Anschlusses an die Ausrichteeinrichtung 4 gewährleistet werden.

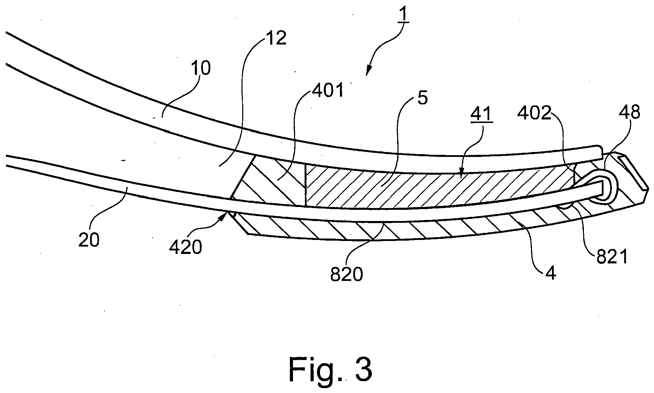

Figur 3 zeigt eine Schnittdarstellung durch den vorderen Teil eines fertig montierten Prothesenfußes als orthopädietechnische Komponente 1 mit einem oberen, auf der Ausrichteeinrichtung 4 aufliegenden ersten Strukturbauteil 10 in Gestalt einer Vorderfußfeder aus einem faserverstärkten Kunststoffmaterial, der Ausrichteeinrichtung 4 und dem in die Ausrichteeinrichtung 4 eingeführten zweiten Strukturbauteil 20 in Gestalt einer Basisfeder, die ebenfalls als Blattfeder aus einem faserverstärkten Kunststoffmaterial ausgebildet ist. Die obere Blattfeder liegt auf einer oberen Auflagefläche auf, die untere Blattfeder auf einer unteren Auflagefläche 820. An dem vorderen, im dargestellten Ausführungsbeispiel rechten Ende der Ausrichteeinrichtung 4 ist ein Kanal 48 ausgebildet, der von der Unterseite des zweiten Strukturbauteils 20 zu dem Hohlraum 41 führt, der von dem zweiten Strukturbauteil 20, dem ersten Strukturbauteil 10 sowie der Ausrichteeinrichtung 4 eingeschlossen wird. In der Auflagefläche 820, die durch die dem zweiten Strukturbauteil 20 zugewandte Oberfläche der Basis der Ausrichteeinrichtung 4 gebildet ist, sind Vertiefungen 821 ausgebildet, so dass auch unterhalb des zweiten Strukturbauteils 20 aufgrund einer strukturierten Oberfläche oder der Vertiefungen 821, die in strömungstechnischer Verbindung mit dem Hohlraum 41 stehen, Klebstoff 5, der den Hohlraum 41 vollständig ausfüllt, in die Vertiefungen 821 eindringen kann, so dass zumindest das untere Strukturbauteil 20 an mehreren Seiten oder an mehreren Stellen von dem Klebstoff 5 umgeben ist. Vorteilhafterweise ist ein Zuführanschluss 44 an der geodätisch tiefsten Stelle der Ausrichteeinrichtung 4 während der Montage angeordnet, in der dargestellten Ausrichtung, beispielsweise an der Unterseite der Ausrichteeinrichtung 4, und steht sowohl mit den Vertiefungen 821 und, aufgrund des Kanals 48, auch mit dem Hohlraum 41 in strömungstechnischer Verbindung. Wird nun an der tiefsten Stelle Klebstoff 5 zugeführt, drückt sich dieser durch die strukturierte Oberfläche auf der Oberseite der Basis der Ausrichteeinrichtung 4 durch die Vertiefungen 821, durch den Kanal 48 in den Hohlraum 41, wobei die bislang darin eingeschlossene Luft durch den nicht dargestellten Auslasskanal abgeführt wird.

Der Figur 3 ist zudem eine Einführöffnung 420 für das zweite Strukturbauteil 20 zu entnehmen, die im dargestellten Ausführungsbeispiel als Schlitz ausgebildet ist und auf der Höhe der Oberseite der Basis, die die Auflagefläche 820 bildet, endet. Oberhalb der Einführöffnung 420 ist ein erster Abstandshalter 401 angeordnet, auf den das erste Strukturbauteil 10 aufgelegt ist, so dass sich zwischen dem ersten Strukturbauteil 10 und dem zweiten Strukturbauteil 20 ein Zwischenraum 12 bildet, der sich auch nach vorne hin fortsetzt, da an dem vorderen Ende ein zweiter Abstandshalter 402 ausgebildet ist, der als Auflagefläche für das erste Strukturbauteil 10 dient. Der Figur 3 ist zu entnehmen, dass die Einführöffnung 420 so bemessen ist, dass die untere Blattfeder dicht anliegend hindurchgedrückt und eingeschoben werden kann. Dadurch wird vermieden, dass beim Einführen des Klebstoffes 5 Klebstoff aus einem Bereich der Einführöffnung 420 um das zweite Strukturbauteil 20 herum austreten kann. Die Dichtwirkung wird durch das Anpressen des ersten Strukturbauteils 10 auf den Abstandshalter 401 und damit auf das zweite Strukturbauteil 20 vergrößert. Aufgrund des zweiten Pressenschuhs 72 liegt die Auflagefläche 820 dicht an dem Strukturbauteil 20 an, so dass kein Klebstoff beim Befüllen des Hohlraums 41 entweichen kann.

Das vordere Ende des unteren Strukturbauteils 20 ist vollständig in der Aufnahmeeinrichtung 4 aufgenommen und wird allseitig geschützt und umgeben, die Einfassung oder Umrandung der oberen Auflagefläche für das erste Strukturbauteil schützt die Blattfeder an dem Umfang, an der Unterseite findet der Schutz durch den Klebstoff sowie die Auflagefläche auf der Ausrichteeinrichtung 4 statt, lediglich die Oberseite ist ungeschützt.

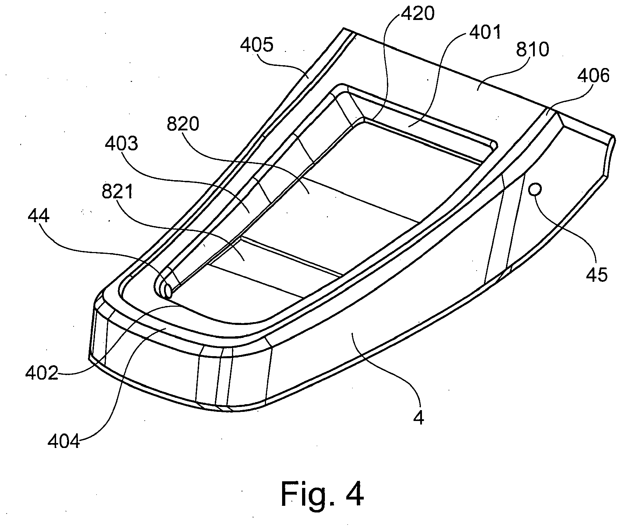

Figur 4 zeigt in einer perspektivischen Darstellung einer Aufnahmeeinrichtung 4 gemäß der Ausführungsform der vorherigen Figuren. Neben dem Zuführanschluss 44, dem Auslasskanal 45 und der unteren Auflagefläche 820 ist die Vertiefung 821 etwas vergrößert dargestellt. Der Kanal 48, der in strömungstechnischer Verbindung mit der Vertiefung 821 steht, ist nicht dargestellt. Es sind die Abstandshalter 401, 402 an der Rückseite und der Vorderseite zu erkennen. Die Abstandshalter 401, 402 bilden an ihren Oberseiten gleichzeitig eine obere Auflagefläche 810 für das nicht dargestellte erste Strukturbauteil aus, das mit seiner Unterseite auf die Auflagefläche 810 gepresst wird. Der Einführschlitz oder die Einführöffnung 420 endet auf der Höhe der unteren Auflagefläche 820. Seitlich neben der Auflagefläche 820 ist in den seitlichen Abstandshaltern 403 eine Nut eingearbeitet, in die das blattförmige Strukturbauteil 20 eingeführt wird, bis es an den vorderen Abschluss der Ausrichteeinrichtung 4 anschlägt.

Die obere Auflagefläche 810 wird von Seitenwänden 404, 405, 406 eingefasst, die in ihrer Materialstärke der des oberen Strukturbauteils 10 entsprechen können. Durch die Seitenwände 404, 405, 406 wird eine definierte Zuordnung des oberen Strukturbauteils 10 zu der Ausrichteeinrichtung 4 und damit zu dem unteren Strukturbauteil 20 gewährleistet, wenn die vorderen und seitlichen Kanten des Strukturbauteils 10 an den jeweiligen Seitenwänden 404, 405, 406 anliegen. Entspricht die Höhe der Seitenwände 404, 405, 406 der Materialstärke des oberen Strukturbauteils 10, kann ein bündiger Abschluss der Oberflächen erreicht werden.

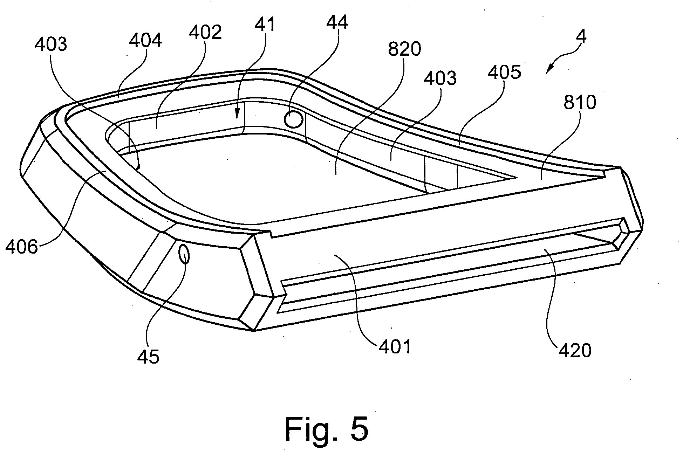

Figur 5 zeigt die Ausrichteeinrichtung 4 gemäß den vorherigen Ausführungsformen in einer Ansicht von schräg hinten, aus der der hintere Abstandshalter 401, der vordere Abstandshalter 402 sowie die Einführöffnung 420 sehr gut zu erkennen sind. Ebenso ist zu erkennen, dass der Zuführanschluss 44 tiefer als der Auslasskanal 45 ausgebildet ist, wobei sowohl der Zuführanschluss 44 als auch der Auslasskanal 45 innerhalb der Abstandshalter 403 ausgebildet sind. Unterhalb der Abstandshalter 403 ausgebildeten Seitenwände ist durch einen Hinterschnitt eine Nut ausgebildet, in der das zweite Strukturbauteil 20 einführbar ist. Die erhöhten Seitenwände 404, 405, 406, die über die obere Auflagefläche 810 hinaus stehen, sind ebenso zu erkennen, wie die im dargestellten Ausführungsbeispiel ebene Auflagefläche 820 auf der Oberseite der Basis der Ausrichteeinrichtung 4. Durch die Seitenwände 402, 403 und den hinteren Abstandshalter 401 wird innerhalb der Ausrichteeinrichtung 4 eine Aufnahme gebildet, die vollständig mit Klebstoff verfüllt werden kann. Durch das Einführen des unteren Strukturbauteils 20 durch die Einführöffnung 420 wird die Einführöffnung 420 geschlossen, so dass die Aufnahme nach dem Einführen des zweiten Strukturbauteils 20 nur oben offen ist. Wird das nicht dargestellte Strukturbauteil 10 auf die obere Auflagefläche 810 aufgelegt, ist der Hohlraum 41 geschlossen. Nach Befüllen des Hohlraums 41 mit dem Klebstoff steht dieser in klebender Verbindung sowohl mit der Ausrichteeinrichtung 4 als auch mit den beiden Strukturbauteilen 10 und 20.

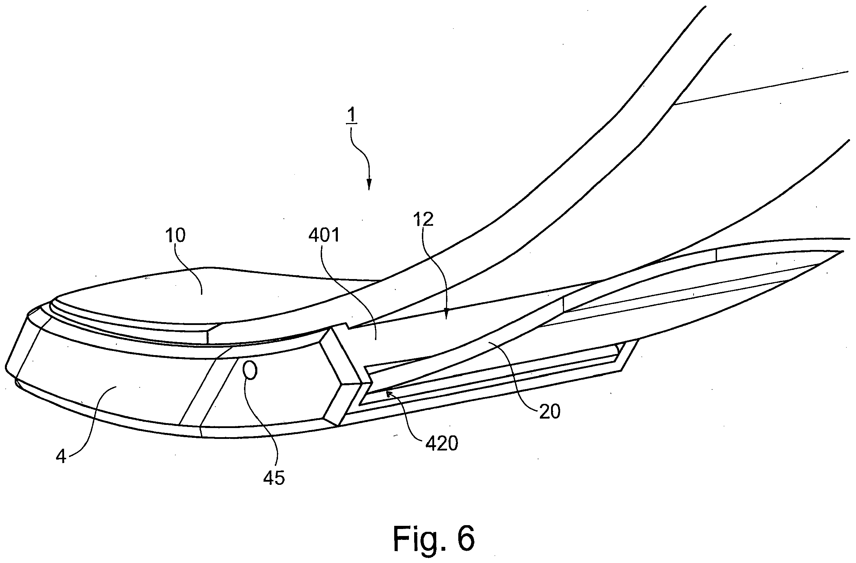

Figur 6 zeigt einen vorderen Teil der orthopädietechnischen Komponente in Gestalt eines Prothesenfußes von schräg hinten in einem fertig montierten Zustand. Das untere Strukturbauteil 20 ist in die Einführöffnung 420 eingeführt, das obere Strukturbauteil 10 ist unter Ausbildung eines Zwischenraumes 12, der durch den Abstandshalter 401 sichergestellt ist, auf der nicht dargestellten Auflagefläche 810 aufgelegt und gehalten. Der Auslasskanal ist in einer Seitenwand angeordnet, die Komponenten 4, 10, 20 sind dauerhaft über den Klebstoff innerhalb der Aufnahmeeinrichtung 4 verbunden.

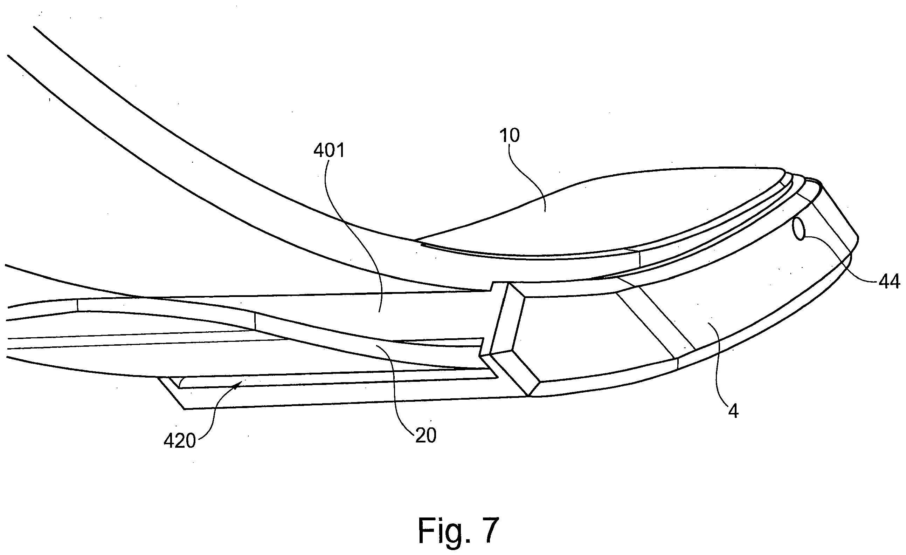

Figur 7 zeigt die Ausführungsform gemäß Figur 6 von der anderen Seite, der Zuführanschluss 44 ist an einer vorderen Seitenwand der Ausrichteeinrichtung 4 angeordnet.

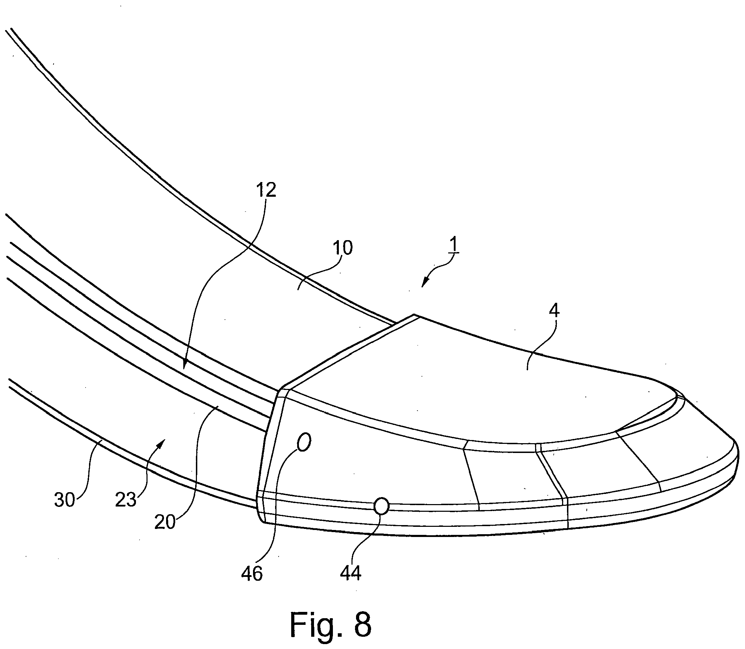

Figur 8 zeigt eine Variante der Erfindung, bei der statt lediglich zweier Strukturbauteile, wie dies in den Figuren 1 bis 7 dargestellt ist, drei Strukturbauteile 10, 20, 30 miteinander über eine Ausrichteeinrichtung 4 verbunden sind. Die orthopädietechnische Komponente 1 ist wiederum als ein Prothesenfuß ausgebildet und weist eine Basisfeder als unteres Strukturbauteil 30 auf. Die Vorfußfeder ist als eine parallel geschaltete, doppelte Blattfederanordnung ausgebildet, die zwei Blattfedern als mittleres Strukturbauteil 20 und oberes Strukturbauteil 10 aufweisen. Die Orientierung der Doppelfeder und der Basisfeder entspricht der Orientierung, wie sie weiter oben beschrieben wurde, grundsätzlich sind auch abweichende Orientierungen und Ausrichtungen der Strukturbauteile 10, 20, 30 zueinander möglich und vorgesehen.

Zwischen dem unteren Strukturbauteil 30 und dem mittleren Strukturbauteil 20 ist ein Zwischenraum 23 ausgebildet, während zwischen dem oberen Strukturbauteil 10 und dem mittleren Strukturbauteil 20 ein zweiter Zwischenraum 12 ausgebildet ist. Der Zwischenraum wird durch entsprechende Abstandshalter innerhalb der Ausrichteeinrichtung 4 ausgebildet.

Die Ausrichteeinrichtung 4 ist im Gegensatz zu der vorherigen Ausführungsform oben geschlossen, das heißt, dass das obere Strukturbauteil 10 nicht auf eine obere Auflagefläche aufgelegt wird, um einen Hohlraum abzuschließen, vielmehr werden alle Strukturbauteile von der rückwärtigen Seite durch Einführöffnungen in die Ausrichteeinrichtung 4 eingeschoben.

Da sich die Beabstandung der jeweiligen Strukturbauteile 10, 20, 30 innerhalb der Ausrichteeinrichtung 4 fortsetzt, werden zumindest zwei Hohlräume innerhalb der Ausrichteeinrichtung 4 gebildet und voneinander getrennt, so dass im dargestellten Ausführungsbeispiel zwei Zuführanschlüsse 44, 46 vorgesehen sind, so dass eine getrennte Befüllung der Hohlräume erfolgen kann. Hierdurch ist es möglich, beispielsweise unterschiedliche Klebstoffe, unterschiedliche Klebstofftemperaturen oder andere Prozessmerkmale zu realisieren, wenn diese prozesstechnisch erforderlich sind.

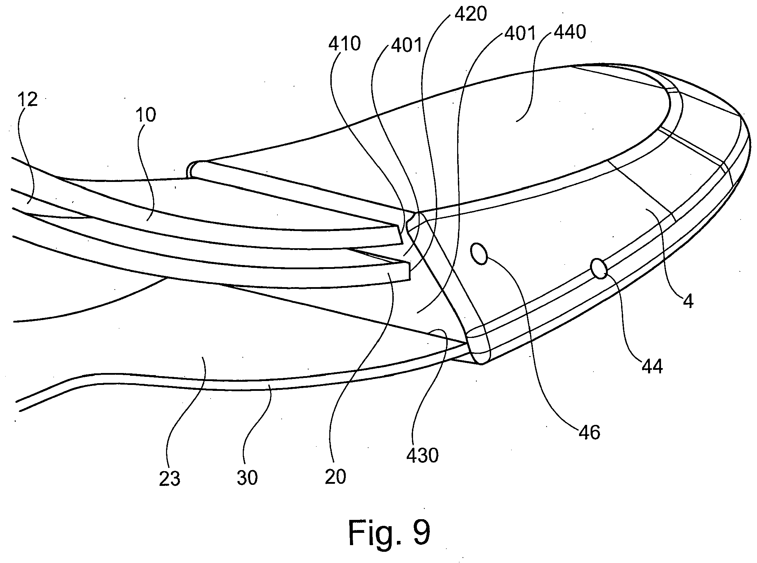

Figur 9 zeigt die Ausführungsform gemäß Figur 8 in einer Ansicht von schräg hinten, Die drei Einführöffnungen 410, 420, 430 an der rückwärtigen Stirnfläche der Ausrichteeinrichtung 4 sind ebenso zu erkennen wie die beiden Zuführanschlüsse 44, 46 und die durch die rückwärtige Wand ausgebildeten hinteren Abstandshalter 401 zwischen den Strukturbauteilen 10, 20, 30.

Die Einführöffnung 430 für das untere Strukturbauteil 30 liegt, wie in der vorherigen Ausführungsform, auf dem Niveau der unteren Auflagefläche 820, auch die Nut, bevorzugt eine umlaufende Nut in der Seitenwand und eine gegebenenfalls vorhandene Strukturierung der Auflagefläche können vorgesehen sein. Statt der oben offenen Ausführung ist in dem dargestellten Ausführungsbeispiel gemäß Figur 9 eine Abdeckung 440 vorgesehen, so dass auch die Oberseite des oberen Strukturbauteils 10 von dem Material der Ausrichteeinrichtung 4 abdeckt wird. Somit sind die vorderen Enden sämtlicher Strukturbauteile 10, 20, 30 vollständig von der Ausrichteinrichtung 4 umgeben und über den Klebstoff miteinander und mit der Ausrichteeinrichtung 4 verbunden.

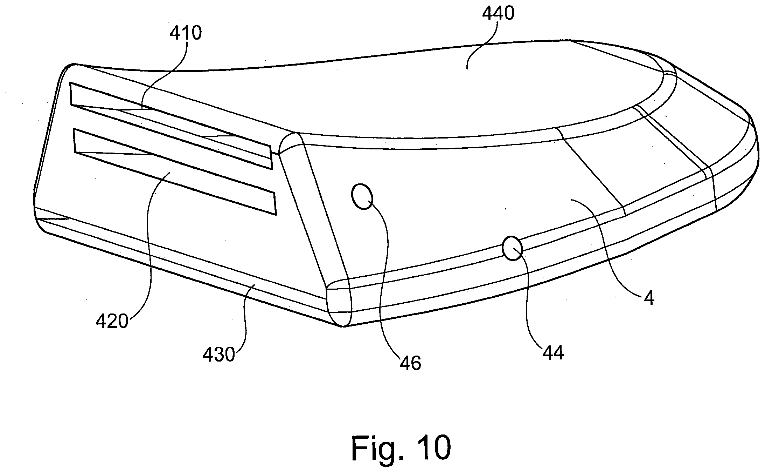

Figur 10 zeigt die Ausrichteeinrichtung 4 gemäß dem zweiten Ausführungsbeispiel in einer Einzeldarstellung. Es sind die drei Einführöffnungen 410, 420, 430 an der Rückseite ebenso zu erkennen, wie die beiden seitlichen Zuführanschlüsse 44, 46, die einen Zugang zu den Zwischenräumen bzw. durch das Einführen der Strukturbauteile 10, 20, 30 entstehenden Hohlräume innerhalb der Ausrichteeinrichtung 4 erlauben. Die obere Abdeckung 440 bildet den oberen Abschluss, die Basis der Ausrichteeinrichtung 4 bildet den unteren Abschluss und eine Art Sohle bei einer Ausgestaltung der orthopädietechnischen Komponente als Prothesenfuß.

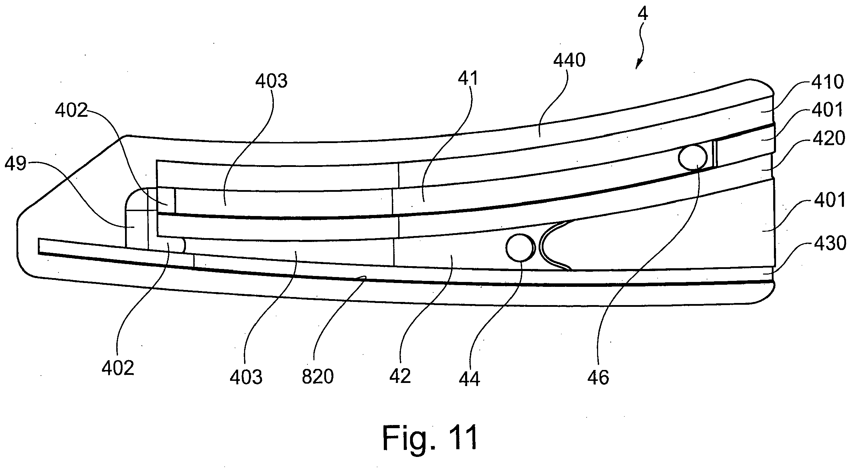

Figur 11 zeigt eine Schnittansicht der Ausrichteeinrichtung 4, aus der die Einführöffnungen 410, 420, 430, die hinteren Abstandshalter 401 sowie die vorderen und seitlichen Abstandshalter 402, 403 zu erkennen sind. Ebenso ist ein Kanal durch die vorderen Abstandshalter 402 hindurchgehend ausgebildet, so dass Klebstoff durch die Zuführanschlüsse 44, 46 seitlich in die Hohlräume 41, 42, die durch die Ausrichteeinrichtung 4 und die darin aufgenommenen Strukturbauteile ausgebildet werden, eingelassen werden kann. Alternativ zu der in den Figuren 8 bis 10 dargestellten Ausführungsform ist es möglich, das nur die untere Öffnung 44 als Zuführanschluss ausgebildet ist, während die obere Öffnung als Auslasskanal ausgebildet ist, so dass Klebstoff durch den Zuführanschluss 44, durch den Hohlraum 42 und den Kanal 49 in den Hohlraum 41 gelangt und dann durch den Auslasskanal austritt. Die Auflagefläche 820 kann strukturiert sein und ebenfalls von Klebstoff umspült oder benetzt werden, so dass das mittlere Strukturbauteil sowohl auf der Unterseite als auch auf der Oberseite von Klebstoff umgeben ist und beidseitig mit je einem anderen Strukturbauteil 10, 30 darauf verbunden ist. Die geschlossene Abdeckung 440 ist ebenso zu erkennen, wie die geschlossene vordere Spitze, eine Einführnut für das untere Strukturbauteil, die über den Kanal 49 in vordere Richtung hinausragt. Die Zuführanschlüsse 44, 46 bzw. der Zuführanschluss 44 und der Auslasskanal sind in den seitlichen Abstandshaltern 403 ausgebildet.

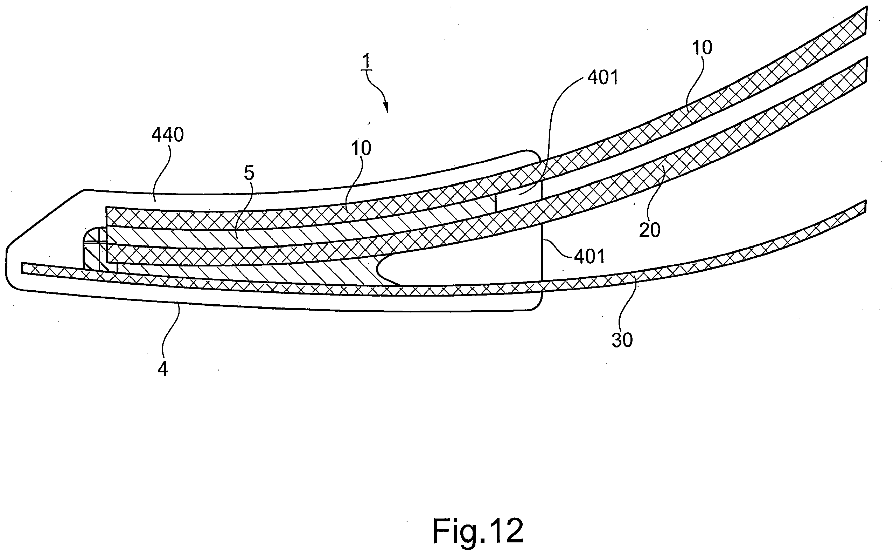

Figur 12 zeigt das vordere Ende der orthopädietechnischen Komponente 1 im montierten Zustand in einer schematischen Schnittdarstellung. Die drei Strukturbauteile 10, 20, 30 in Gestalt von Blattfedern sind durch die jeweiligen Einführöffnungen in die Ausrichteeinrichtung 4 eingeführt und die hinteren Abstandshalter 401, die nicht dargestellten Abstandshalter 403 und die vorderen Abstandshalter 402 zueinander beabstandet in der Ausrichteinrichtung 4 gehalten. Der Klebstoff 5 ist durch den nicht dargestellten Zuführanschluss 44 in den Hohlraum 41 eingeführt, durch den Kanal 49 in den oberen Hohlraum 42 eingedrungen und durch den nicht dargestellten oberen Auslasskanal 45 abgeführt worden. Durch den dichtenden Abschluss der Einführöffnungen 410, 420, 430 um die Strukturbauteile 10, 20, 30 herum ist kein Klebstoff 5 während der Fertigung rückwärtig ausgetreten. Der Klebstoff umgibt das zweite Strukturbauteil 20 an der Oberseite, an der Vorderseite sowie an der Unterseite.

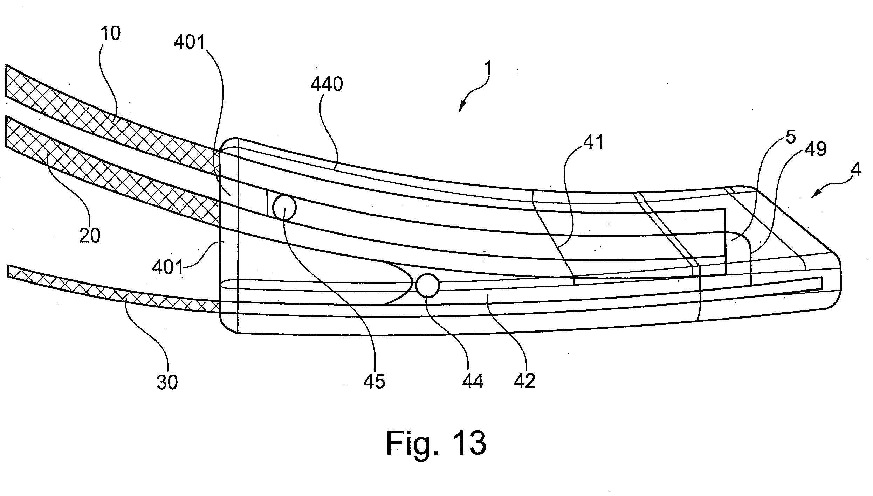

Figur 13 zeigt eine Seitenansicht des montierten Prothesenfußes bzw. der orthopädietechnischen Komponente 1, bei der statt zweier Zuführanschlüsse ein unterer Zuführanschluss 44 und ein oberer Auslasskanal 45 in der Ausrichteeinrichtung 4 vorgesehen sind. Die drei eingeführten Strukturbauteile 10, 20, 30 sind ebenso zu erkennen, wie die hinteren Abstandshalter 401, die Zwischenräume oder Hohlräume 41, 42, die nach hinten durch die eingeführten Strukturbauteile 10, 20, 30 abgedichtet werden und die obere Abdeckung 440, durch die auch die obere Blattfeder oder das obere Strukturbauteil 10 vollständig von der Ausrichteinrichtung 4 abgedeckt und geschützt wird.

Der Klebstoff wird durch den Zuführanschluss 44 in den unteren Hohlraum 42 durch den Kanal 49 in den oberen Hohlraum 41 und durch den Auslasskanal 45 hinausgedrückt, sobald Klebstoff aus dem oben gelegenen Auslasskanal 45 austritt, wird die Zufuhr des Klebstoffes durch den Zuführanschluss 44 gestoppt, die Strukturbauteile 10, 20, 30 in der gewünschten Zuordnung gehalten und gewartet, bis der Klebstoff ausgehärtet ist, so dass sämtliche Komponenten 10, 20, 30, 4 dauerhaft miteinander verbunden sind.

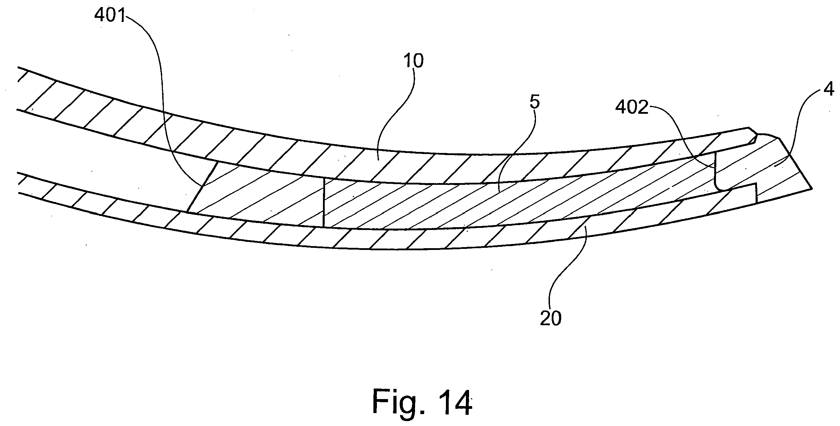

Figur 14 zeigt eine weitere Variante der Erfindung, bei der die Ausrichteeinrichtung 4 ohne untenseitigen Boden ausgebildet ist. Die Ausrichteeinrichtung 4 ist hier als ein Rahmen mit Auflageflächen für die oben und unten aufgelegten Strukturbauteile 10, 20 ausgebildet. Der Rahmen ist umlaufend mit einer eingeschlossenen Öffnung, die durch die Strukturbauteile 10, 20 zu einem Hohlraum komplettiert wird, in den Klebstoff 5 eingeführt wird, so dass die Unterseite des oberen Strukturbauteils 10 und die Oberseite des unteren Strukturbauteils 20 einander gegenüberliegend mit Klebstoff 5 benetzt und miteinander an der Ausrichteeinrichtung 4 verklebt werden. Beide Strukturbauteile 10, 20 werden auf die jeweilige Auflagefläche gedrückt und gedrückt gehalten, bis der Klebstoff 5 ausgehärtet ist, durch das Andrücken auf die Auflageflächen wird der gebildete Hohlraum abgedichtet, überschüssiger Klebstoff 5 tritt nur durch den in einem Abstandshalter angeordneten Auslasskanal, vorzugsweise über eine Auslasseinrichtung aus, so dass das Bauteil nicht mit Klebstoff 5 verunreinigt wird.

Durch das erfindungsgemäße Verfahren und die erfindungsgemäße orthopädietechnische Komponente ist es möglich, zwei Strukturbauteile, insbesondere zwei Faserverbundwerkstoffe mit einem flüssigen Klebstoff zu verkleben und diese Strukturbauteile gleichzeitig zu umgeben, um dadurch eine schützende Umhüllung bereitzustellen. Die Ausrichteeinrichtung passt an oder auf die zu verbindenden Komponenten und bildet eine Kavität zwischen ihnen aus, die den Aufnahmeraum für den flüssigen Klebstoff bildet. Um den Klebstoff in die Kavität oder den Hohlraum einzuführen und gleichzeitig die Kavität zu entlüften, sind vergleichsweise kleine Öffnungen in Gestalt von Zuführkanälen oder Auslasskanälen in die Ausrichteinrichtung oder die Form und Umhüllung integriert. In diese Zuführanschlüsse und Auslasskanäle können Schlauchverbinder eingesetzt werden, die mit einem Zuführschlauch und einem Entlüftungsschlauch verbunden werden können. Um sicherzustellen, dass der Hohlraum oder die Kavität sicher abgedichtet ist, können die Strukturbauteile zusammengepresst oder gegen die Aufnahmeeinrichtung 4 gepresst werden, wobei dies durch flexible Materialien geschehen kann. Vorzugsweise ist das Material der Ausrichteeinrichtung 4 ein flexibles, elastisches Material, so dass eine abdichtende Anlage an den Strukturbauteilen durch Ausüben von Druck in Richtung auf die Strukturbauteile gewährleistet werden kann. Nach dem Einführen des Klebstoffes und dem Aushärten werden die Schlauchverbinder von der Ausrichteeinrichtung oder der Formenhülle entfernt und das Verbindungsverfahren ist abgeschlossen. Die Form dient nun nicht mehr als Begrenzung für den Klebstoff, sondern wird als Hülle oder Umhüllung der Strukturbauteile eingesetzt, um die Strukturbauteile gegen eine Beschädigung zu schützen und darüber hinaus um weitere Bauteile, beispielsweise eine Umhüllung oder eine Kosmetik vor Schäden durch die miteinander verbundenen Strukturbauteile, die scharfkantig sein können, zu schützen.

Durch die Vorrichtung und das Verfahren ist es möglich, dass eine Form für einen flüssigen Klebstoff zum Verbinden zweier Strukturbauteile bereitgestellt wird. Die Ausrichtung der zu verbindenden Komponenten ist durch die Ausrichteinrichtung 4 gewährleistet, ebenso werden die zu verbindenden Bauteile geschützt, das Herstellverfahren ist sauber und eine Nachbearbeitung der Klebestelle ist nicht notwendig. Der Verbrauch von Klebstoff wird eingeschränkt, da kein überschüssiger Klebstoff austreten kann und ein definiertes Volumen durch die jeweiligen Hohlräume als Grundlage für die Berechnung der zugeführten Klebstoffmenge dienen kann. So ist durch ein mengengesteuertes Zuführen von Klebstoff sichergestellt, dass einerseits eine minimale Klebstoffmenge genutzt und andererseits stets ausreichend Klebstoff zum vollständigen Ausfüllen des Hohlraumes bereitgestellt wird.

**Bezugszeichenliste**

| | | | |
|---|---|---|---|
| 1 | orthopädietechnische Komponente | 810 | Auflagefläche |
| 4 | Ausrichteeinrichtung | 820 | Auflagefläche |
| 5 | Klebstoff | 821 | Vertiefung |
| 7 | Presse | | |
| 10 | Strukturbauteil | | |
| 12 | Zwischenraum | | |
| 20 | Strukturbauteil | | |
| 23 | Zwischenraum | | |
| 30 | Strukturbauteil | | |
| 41 | Hohlraum | | |
| 42 | Hohlraum | | |
| 44 | Zuführanschluss | | |
| 45 | Auslasskanal | | |
| 46 | Zuführanschluss | | |
| 47 | Auslasskanal | | |
| 48 | Kanal | | |
| 49 | Kanal | | |
| 51 | Zuführeinrichtung | | |
| 52 | Auslasseinrichtung | | |
| 71 | Pressenschuh | | |
| 72 | Pressenschuh | | |
| 401 | Abstandshalter | | |
| 402 | Abstandshalter | | |
| 403 | Abstandshalter | | |
| 404 | Seitenwände | | |
| 405 | Seitenwände | | |
| 406 | Seitenwände | | |
| 410 | Einführöffnungen | | |
| 420 | Einführöffnungen | | |
| 430 | Einführöffnungen | | |
| 440 | Abdeckung | | |

## Patentansprüche

1. Verfahren zum Verbinden zumindest zweier Strukturbauteile (10, 20, 30) einer orthopädietechnischen Komponente (1), bei dem die Strukturbauteile (10, 20, 30) in einer Ausrichteeinrichtung (4) unter Ausbildung eines Zwischenraumes (12, 23) zwischen den Strukturbauteilen (10, 20, 30) zueinander orientiert gehalten werden, wobei die Ausrichteeinrichtung (4) und die Strukturbauteile (10, 20, 30) gemeinsam einen Hohlraum (41, 42) ausbilden, der mit zumindest einem Zuführanschluss (44, 46) in strömungstechnischer Verbindung steht und über den ein Klebstoff (5) zum Verkleben der Strukturbauteile (10, 20, 30) in dem Hohlraum (44, 46) eingeführt wird, **dadurch gekennzeichnet, dass** die Ausrichteeinrichtung (4) nach dem Verkleben nicht von den Strukturbauteilen (10, 20, 30) getrennt wird.

2. Verfahren nach Anspruch 1, **dadurch gekennzeichnet, dass** die Strukturbauteile (10, 20, 30) zueinander orientiert gehalten werden, bis der Klebstoff (5) ausgehärtet ist.

3. Verfahren nach Anspruch 1 oder 2, **dadurch gekennzeichnet, dass** zumindest ein Strukturbauteil (10, 20, 30) in dem Klebstoff (5) eingebettet wird.

4. Verfahren nach einem der voranstehenden Ansprüche, **dadurch gekennzeichnet, dass** zumindest ein Strukturbauteil (10, 20, 30) gegen die Ausrichteeinrichtung (4) gepresst wird.

5. Verfahren nach einem der voranstehenden Ansprüche, **dadurch gekennzeichnet, dass** der Hohlraum (41, 42) durch das Halten der Strukturbauteile (10, 20, 30) mit Ausnahme des Zuführanschlusses (44, 46) und eines Auslasskanals (45, 47) abgedichtet wird.

6. Verfahren nach einem der voranstehenden Ansprüche, **dadurch gekennzeichnet, dass** der Klebstoff (5) über zumindest eine Zuführeinrichtung (51) in den Hohlraum (44, 46) eingeführt wird, die zumindest eine Zuführeinrichtung (51) bis zum Aushärten des Klebstoffes (5) an der Ausrichteeinrichtung (4) verleibt und anschließend entfernt wird.

7. Orthopädietechnische Komponente mit zumindest zwei Strukturbauteilen (10, 20, 30), die zueinander beabstandet miteinander verklebt sind, wobei die Strukturbauteile (10, 20, 30) in einer Ausrichteeinrichtung (4) zueinander orientiert gehalten sind und die Ausrichteeinrichtung (4) Abstandhalter (401, 402, 403) aufweist, die die Strukturbauteile (10, 20, 30) voneinander beabstanden, **dadurch gekennzeichnet dass**, die Ausrichteeinrichtung (4) mit den Strukturbauteilen (10, 20, 30) verklebt ist.

8. Orthopädietechnische Komponente nach Anspruch 7, **dadurch gekennzeichnet, dass** die Ausrichteeinrichtung (4) zumindest eine Einführöffnung (410, 420, 430) für zumindest ein Strukturbauteil (10, 20, 30) aufweist und die Form der Einführöffnung (4190, 420, 430) der Kontur des Strukturbauteils (10, 20, 30) einspricht.

9. Orthopädietechnische Komponente nach Anspruche 7 oder8 **dadurch gekennzeichnet, dass** die Strukturbauteile (10, 20, 30) als Blattfedern ausgebildet sind.

10. Orthopädietechnische Komponente nach einem der Ansprüche 7 bis 9, **dadurch gekennzeichnet, dass** in der Ausrichteeinrichtung (4) zumindest ein Verbindungskanal (48, 49) ausgebildet ist, der zwei voneinander durch ein Strukturbauteil (10, 20, 30) getrennte Hohlräume (41, 42) miteinander verbindet.

11. Orthopädietechnische Komponente nach einem der Ansprüche 7 bis 10, **dadurch gekennzeichnet, dass** sie als Prothesenfuß oder Orthesenkomponente ausgebildet ist.

12. Orthopädietechnische Komponente nach einem der Ansprüche 7 bis 11, **dadurch gekennzeichnet, dass** die Ausrichteeinrichtung (4) an zumindest einem Strukturbauteil (10, 20, 30) an zumindest drei Seiten anliegt oder es umgibt.

13. Orthopädietechnische Komponente nach einem der Ansprüche 7 bis 12, **dadurch gekennzeichnet, dass** das Strukturbauteil (10, 20, 30) als faserverstärktes Kunststoffbauteil ausgebildet ist.

14. Orthopädietechnische Komponente nach einem der Ansprüche 7 bis 13, **dadurch gekennzeichnet, dass** die Ausrichteeinrichtung (4) als funktionale Komponente ausgebildet ist.

15. Orthopädietechnische Komponente nach einem der Ansprüche 7 bis 14, **dadurch gekennzeichnet, dass** der Kleber (4) aus einem elastischen Material besteht, insbesondere aus einem Polyurethan.

## Claims

1. A method for connecting at least two structural parts (10, 20, 30) of an orthopedic component (1), in which the structural parts (10, 20, 30) are retained in an orienting device (4) while oriented in relation to each other with an intermediate space (12, 23) thus being formed between the structural parts (10, 20, 30), wherein the orienting device (4) and the structural parts (10, 20, 20) together form a hollow space (41, 42), which is fluidically connected to at least one feed connection (44, 46) and via which an adhesive (5) for adhesively bonding the structural parts (10, 20, 30) is introduced into the hollow space (44, 46) **characterized in that** the orienting device (4) is not separated from the structural parts (10, 20, 30) after the adhesive bonding.

2. The method as claimed in claim 1, **characterized in that** the structural parts (10, 20, 30) are held oriented in relation to one another until the adhesive (5) has cured.

3. The method as claimed in claim 1 or 2, **characterized in that** at least one structural part (10, 20, 30) is embedded in the adhesive (5).

4. The method as claimed in any one of the preceding claims, **characterized in that** at least one structural part (10, 20, 30) is pressed against the orienting device (4).

5. The method as claimed in any one of the preceding claims, **characterized in that** the hollow space (41, 42) is sealed off with the exception of the feed connection (44, 46) and an outlet channel (45, 47) by holding the structural parts (10, 20, 30).

6. The method as claimed in any one of the preceding claims, **characterized in that** the adhesive (5) is introduced into the hollow space (44, 46) via at least one feed device (51), the at least one feed device (51) remains on the orienting device (4) until the adhesive (5) is cured, and is then removed.

7. An orthopedic component having at least two structural parts (10, 20, 30), which are adhesively bonded to one another at a distance from one another, wherein the structural parts (10, 20, 30) are held in an orienting device (4) oriented in relation to one another, the orienting device (4) has spacers (401, 402, 403) which distance the structural parts (10, 20, 30) from one another, **characterized in that** the orienting device (4) is adhesively bonded to the structural parts (10, 20, 30).

8. The orthopedic component as claimed in claim 7, **characterized in that** the orienting device (4) has at least one insertion opening (410, 420, 430) for at least one structural part (10, 20, 30) and the shape of the insertion opening (410, 420, 430) corresponds to the contour of the structural part (10, 20, 30).

9. The orthopedic component as claimed in claim 7 or 8, **characterized in that** the structural parts (10, 20, 30) are formed as leaf springs.

10. The orthopedic component as claimed in any one of claims 7 to 9, **characterized in that** at least one connection channel (48, 49) is formed in the orienting device (4) and connects two hollow spaces (41, 42) separated from one another by a structural part (10, 20, 30) to one another.

11. The orthopedic component as claimed in any one of claims 7 to 10, **characterized in that** it is formed as a prosthetic foot or orthotic component.

12. The orthopedic component as claimed in any one of claims 7 to 11, **characterized in that** the orienting device (4) bears against or surrounds at least one structural part (10, 20, 30) on at least three sides.

13. The orthopedic component as claimed in any one of claims 7 to 12, **characterized in that** the structural part (10, 20, 30) is formed as a fiber-reinforced plastics component.

14. The orthopedic component as claimed in any one of claims 7 to 13, **characterized in that** the orienting device (4) is formed as a functional component.

15. The orthopedic component as claimed in any one of claims 7 to 14, **characterized in that** the adhesive (4) consists of a resilient material, in particular of a polyurethane.

## Revendications

1. Procédé pour relier au moins deux composants de structure (10, 20, 30) d'un ensemble orthopédique (1), dans lequel les composants de structure (10, 20, 30) sont retenus de façon orientée les uns par rapport aux autres dans un moyen d'orientation (4) en formant un intervalle (12, 23) entre les composants de structure (10, 20, 30), le moyen d'orientation (4) et les composants de structure (10, 20, 30) réalisant conjointement une cavité (41, 42) qui est en communication fluidique avec au moins un raccord d'alimentation (44, 46) par lequel une colle (5) pour coller les composants de structure (10, 20, 30) est introduite dans la cavité (44, 46),
**caractérisé en ce que**
après le collage, le moyen d'orientation (4) n'est pas séparé des composants de structure (10, 20, 30).

2. Procédé selon la revendication 1,
**caractérisé en ce que**
les composants de structure (10, 20, 30) sont retenus de façon orientée les uns par rapport aux autres jusqu'à ce que la colle (5) soit durcie.

3. Procédé selon la revendication 1 ou 2,
**caractérisé en ce que**
au moins un composant de structure (10, 20, 30) est noyé dans la colle (5).

4. Procédé selon l'une des revendications précédentes,
**caractérisé en ce que**
au moins un composant de structure (10, 20, 30) est pressé contre le moyen d'orientation (4).

5. Procédé selon l'une des revendications précédentes,
**caractérisé en ce que**
la cavité (41, 42) est étanchée grâce à la retenue des composants de structure (10, 20, 30), exception faite du raccord d'alimentation (44, 46) et d'un canal de sortie (45, 47).

6. Procédé selon l'une des revendications précédentes,
**caractérisé en ce que**
la colle (5) est introduite dans la cavité (44, 46) par au moins un moyen d'alimentation (51), ledit au moins un moyen d'alimentation (51) demeurant sur le moyen d'orientation (4) jusqu'à la colle (5) soit durcie, et ensuite il est enlevé.

7. Ensemble orthopédique comportant deux composants de structure (10, 20, 30) qui sont collés les uns aux autres et à distance les uns des autres, les composants de structure (10, 20, 30) étant retenus de façon orientée les uns par rapport aux autres dans un moyen d'orientation (4), et le moyen d'orientation (4) comprenant des écarteurs (401, 402, 403) qui écartent les composants de structure (10, 20, 30) les uns des autres,
**caractérisé en ce que**
le moyen d'orientation (4) est collé aux composants de structure (10, 20, 30).

8. Ensemble orthopédique selon la revendication 7,
**caractérisé en ce que**
le moyen d'orientation (4) présente au moins une ouverture d'introduction (410, 420, 430) pour au moins un composant de structure (10, 20, 30) et la forme de l'ouverture d'introduction (410, 420, 430) correspond au contour du composant de structure (10, 20, 30).

9. Ensemble orthopédique selon la revendication 7 ou 8,
**caractérisé en ce que**
les composants de structure (10, 20, 30) sont réalisés sous forme de ressorts à lame.

10. Ensemble orthopédique selon l'une des revendications 7 à 9,
**caractérisé en ce que**
au moins un canal de liaison (48, 49) est réalisé dans le moyen d'orientation (4), canal qui relie l'une à l'autre deux cavités (41, 42) qui sont séparées par un composant de structure (10, 20, 30).

11. Ensemble orthopédique selon l'une des revendications 7 à 10,
**caractérisé en ce que**
il est réalisé sous forme de prothèse de pied ou d'ensemble d'orthèse.

12. Ensemble orthopédique selon l'une des revendications 7 à 11,
**caractérisé en ce que**
le moyen d'orientation (4) s'appuie contre ou entoure au moins un composant de structure (10, 20, 30) sur au moins trois côtés.

13. Ensemble orthopédique selon l'une des revendications 7 à 12,
**caractérisé en ce que**
le composant de structure (10, 20, 30) est réalisé sous forme de pièce en matière plastique renforcée de fibres.

14. Ensemble orthopédique selon l'une des revendications 7 à 13,
**caractérisé en ce que**
le moyen d'orientation (4) est réalisé sous forme d'ensemble fonctionnel.

15. Ensemble orthopédique selon l'une des revendications 7 à 14,
**caractérisé en ce que**
la colle (4) est constituée en un matériau élastique, en particulier en un polyuréthane.
